# EUROPEAN PATENT SPECIFICATION

(11) **EP 1 997 532 B1**
(45) Date of publication and mention of the grant of the patent: **30.12.2009**
(21) Application number: 08251844.0
(22) Date of filing: 27.05.2008
(51) Int. Cl.: A61N 5/10, A61M 36/00

(54) **Ophthalmic applicator for treatment of pterygium or glaucoma using 32-P in combination with 103-Pd**
Ophthalmischer Applikator zur Behandlung von Pterygium oder Glaukom mit 32-P in Kombination mit 103-Pd
Applicateur ophtalmique pour le traitement de ptérygion ou glaucome utilisant le 32-P en combinaison avec le 103-Pd

(30) Priority: 28.05.2007 KR 20070051570
(43) Date of publication of application: 03.12.2008
(73) Proprietor: Seoul National University Hospital, Seoul 110-744 (KR); Korea Atomic Energy Research Institute, Daejeon 305-353 (KR)
(72) Inventor: Ye, Sung-Joon, Yongin-si, Gyeonggi-do 448-981 (KR); Kim, Ilhan, Seoul 135-280 (KR); Wee, Won Ryang, Seoul 135-110 (KR); Kim, Mee Kum, Goyang-si, Gyeonggi-do 410-742 (KR); Son, Kwang Jae, Daejon 305-769 (KR); Han, Hyon Soo, Daejeon 305-390 (KR); Park, Ul Jae, Daejeon 305-769 (KR); Shin, Hyeon Young, Daejeon 305-755 (KR)
(74) Representative: Brady, Paul Andrew

(56) References cited:
- WO-A-2004/098523
- US-A- 2 517 568
- US-A- 2 559 793
- US-A1- 2002 115 902
- GLECKLER MARK ET AL: "Calculating lens dose and surface dose rates from 90Sr ophthalmic applicators using Monte Carlo modeling" MEDICAL PHYSICS, AIP, MELVILLE, NY, US, vol. 25, no. 1, 1 January 1998 (1998-01-01), pages 29-36, XP012010304 ISSN: 0094-2405
- JOCHEN WILLNER ET AL: "Soft X-Ray Therapy of Recurrent Pterygium an Alternative to 90Sr Eye Applicators = Röntgennahbestrahlung bei rezidivierenden Pterygien Eine Alternative zu 90Sr-Augenapplikatoren" STRAHLENTHERAPIE UND ONKOLOGIE, URBAN UND VOGEL, MUENCHEN, DE, vol. 177, no. 8, 1 August 2001 (2001-08-01), pages 404-409, XP007905707 ISSN: 0179-7158
- YANG KYUN PARK ET AL: "Potential use of P-32 ophthalmic applicator: Monte Carlo simulations for design and dosimetry" MEDICAL PHYSICS, AIP, MELVILLE, NY, US, vol. 35, no. 5, 17 April 2008 (2008-04-17), pages 1854-1858, XP007905706 ISSN: 0094-2405 [retrieved on 2008-04-17]

## Description

### BACKGROUND OF THE INVENTION

### 1. Field of the Invention

The present invention relates to an ophthalmic applicator for treating pterygium or glaucoma using ³²P in combination with ¹⁰³Pd.

### 2. Description of the Related Art

A pterygium is a wedge-shaped fibrovascular growth of conjunctiva (the surface tissue of the white of the eye) that extends onto and invades the surface of the cornea. In addition to imparting a poor appearance, the growth of a pterygium can obscure vision if it encroaches on the pupil of the eye.

Although the causes of pterygium have remained unclear, hereditary predisposition, along with irritation with UV light, wind or dust are known to significantly contribute to the formation and progression of pterygium.

For the symptomatic therapy of small pterygium, ophthalmologists may recommend a trial of a decongestant, anti-inflammatory eye drops, or the like, but these medications are not a fundamental solution to pterygium.

In some cases, for example, where a pterygium is growing far enough onto the cornea to threaten the line of vision, surgical removal of the tissue may be recommended. Surgical removal is usually completed within 20 - 30 min after local anesthesia, and the patient should remain under the care of the ophthalmologist for about one month in order to ensure the subsidence of pain and a foreign body sensation caused by the operation.

Simple as it is, this surgery frequently entails the disadvantage of recurrence. Even when the surgical operation is successful, the recurrence rate is 30% on average, and as high as 70% in some cases.

Various techniques, such as conjunctival autograft transplantation, chemotherapy, etc., have been used to prevent the recurrence of pterygium post surgery. Like skin autografts, conjunctival autograft transplantation is a method in which a healthy conjunctival graft obtained from a patient is applied to a pterygium-excised site of the same patient. Chemotherapy involves the use of chemicals to prevent the recurrence of pterygium. However, these therapies are still unsatisfactory with regard to pterygium recurrence.

Recently, intensive attention has been paid to radiotherapy for preventing pterygium recurrence.

Since 1920, radiotherapy with radioisotopes has been medically used. In Korea, radiotherapy was first conducted by applying ¹³¹I to a hyperthyroidism patient when the Atomic Energy Act was established in March, 1959. Since then, with the great advances in nuclear medicine, radiotherapy has gradually progressed. Demands for radioisotopes that play a pivotal role in radiotherapy and for therapeutics using radioisotopes increase by 5% and 10% each year, respectively. ¹³¹I accounts for as much as 30% of the total demand for therapeutic radioisotopes. Currently, other isotopes including ⁹⁰Y and ¹⁸⁸Re are under development for use in radiotherapy. Supported by the Korean government's policy of spreading medical cyclotrons over the nation, the medical industry of Korea has become able to produce various therapeutic radioisotopes, such as ²⁰¹TI, ¹²³I, ⁶⁷G, ¹¹¹In, ⁵⁷CO, ¹⁰³Pd in amounts that ensure self-sufficiency. In addition, extensive research into the development of radioisotope therapeutics using β-radiation is now being conducted.

A radioisotope is a version of a chemical element that has an unstable nucleus and emits radiation during its decay into a stable form. Typically, radioisotopes emit three kinds of radiation: alpha radiation, beta radiation and gamma radiation. Elements emitting alpha radiation are highly toxic to the body, in addition to being somewhat difficult to obtain. [On the other hand, gamma radiation, due to its high energy content, can cause serious damage when absorbed by living cells.]

On the other hand, gamma radiation, due to its high penetrative power, often is used in treatment of deep-seated tumor. But it can also cause serious damage when absorbed by normal tissue.

Beta radiation is weakly penetrative, but highly destructive, and thus radioisotopes emitting beta radiation are usually used in radiotherapy for superficial tumors because that can be focused on target lesions with little influence on other healthy parts.

Examples of radioisotopes emitting beta radiation useful in non-sealed therapy include ⁸⁹Sr, ⁹⁰Y, ¹⁸⁸Re, ¹⁵³Sm and ¹⁶⁶Ho.

[Particularly, these radioisotopes decay to their respective stable forms in a short time due to their short half lives, so that they can be focused on target lesions with little influence on other healthy parts.]

Particularly, these radioisotopes decay to their respective stable forms in a short time due to their short half lives so that the radioisotopes can accumulate in target sites only during treatment, without leakage or damage to other sites. The products remaining after decay can be assimilated, degenerated and discharged from the body.

For the application of therapeutic radioisotopes to target lesions, proper account must be taken of many factors including emission properties, physical half life, radiochemical purity, attenuation properties of labeling with high specific radioactivity, ease of production, cost, and convenience of storage and use. In order to increase therapeutic efficiency, first of all, radioisotopes, which carry out therapeutic functions by carrying energy through particle emission, are required to be accumulated in the highest possible amounts in target lesions and in the lowest possible amounts in parts other than target lesions.

It has been confirmed that single-dose beta-irradiation (RT) after bare sclera surgery is a simple, effective, and safe treatment that reduces the risk of primary pterygium recurrence [Jurgenliemk-Schulz IM, Hartman LJ, Roesink JM, Tersteeg RJ, van Der Tweel I, Kal HB, Mourits MP, Wyrdeman HKInt J Radiat Oncol Biol Phys. 2004 Jul 15;59(4):1138-47]. A pure beta-emitter of ⁹⁰Sr has been almost exclusively used for this purpose. Particularly, high-energy beta radiation of ⁹⁰Sr (maximum 2.27 MeV), which is usually used in the treatment of pterygium, can deliver therapeutic doses to the cornea within 1 mm from the applicator. However, ⁹⁰Sr is an isotope that requires heavy radiochemical processing for its production from fission fragments. Furthermore, its long half-life (28.8 yrs) requires additional caution for the production, storage, and disposition thereof.

It is reported that possibility of failure for operation may be remarkably lowered by irradiating beta-radiation of ⁹⁰Sr after having a operation for glaucoma. [J. F. Kirwan, S. C. Cousens, L. Venter, C. Cook, A. Stulting, P. Roux, and I. Murdoch, "Effect of b radiation on success of glaucoma drainage surgery in South Africa: randomized controlled trial," BMJ. 333, 942-946 (2006)]

Medical Physics, 1998, vol. 25, no. 1, p29-36, Gleckler *et al*, describes the calculation of lens dose and surface dose rates from ⁹⁰Sr ophthalmic applicators using Monte Carlo modelling.

WO2004/098523 describes an ocular brachytherapy device, generally comprising a catheter and wire, impregnated with radioactive material. The wire is formed having a desired treatment shape and size such that it can be placed near an area requiring treatment and effectuate treatment while not affecting adjacent areas.

US2002/0115902 describes a surgical device for localized delivery of beta radiation in surgical procedures, particularly ophthalmic procedures. Preferred surgical devices described in that document include a cannula with a beta radiotherapy emitting material at the distal end of the cannula.

US2559793 relates to the use of pure beta irradiation for therapeutic purposes, and more particularly for ophthalmic purposes. It describes an ophthalmic applicator which has radioactive material which consists substantially wholly of radium D and radium E and radium F, said applicator having filter means which are permeable substantially only to beta rays emitted at the energy level of the beta rays emitted by radium E.

US2517568.relates to therapeutic applicators and more particularly to an improved applicator capable of being used uniformly to expose a surface of the human body such as the eyeball to radiations from radioactive substances.

Strahlentherapie und Onkologie, 2001, vol. 177, no. 8, p404-409, Willner *et al*, describes an analysis of the effectiveness of perioperative 20 kV soft X-ray irradiation in recurrent pterygium as an alternative to postoperative ⁹⁰Sr beta irradiation.

Leading to the present invention, intensive and thorough research into a radiation emitter that can replace an ophthalmic applicator using ⁹⁰Sr, conducted by the present inventors, resulted in the finding that the pure-irradiation of ³²P can be used as an alternative to ⁹⁰Sr-irradiation and can deliver more effective therapeutic doses to lesions within a short time, and that the use of ³²P in combination with ¹⁰³Pd, which is a radioactive isotope emitting low-energy photons with a half-life similar to that of ³²P, can provide a uniform dose distribution in the target and the sharp fall-off beyond the target.

### SUMMARY OF THE INVENTION

Accordingly, the present invention has been made keeping in mind the above problems occurring in the prior art, and an object of the present invention is to provide an ophthalmic applicator for the treatment of pterygium or glaucoma using ³²P in combination with ¹⁰³Pd.

In order to accomplish the object, the present invention provides an applicator for the treatment of pterygium or glaucoma, comprising a source volume for containing a radioisotope; a filter that functions to control the radiation dose emitted from the radioisotope; and an encapsulator that encompasses the source volume and the filter, wherein the radioisotope is pure ³²P or a combination of ³²P and ¹⁰³Pd, and wherein the ³²P is responsible for 80~90% of the total radiation dose while the ¹⁰³Pd is responsible for 20~10% of the total radiation dose, correspondingly.

### BREIF DESCRIPTION OF THE DRAWINGS

The above and other objects, features and other advantages of the present invention will be more clearly understood from the following detailed description taken in conjunction with the accompanying drawings, in which:

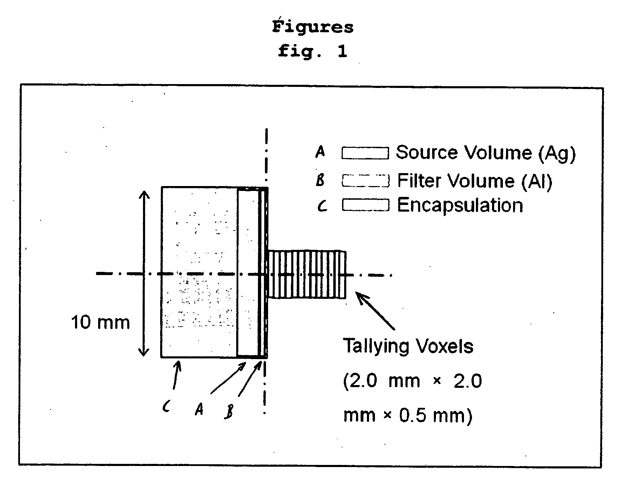

FIG. 1 is a schematic view of an ophthalmic applicator designed on the basis of Monte Carlo simulations;
FIG. 2 is a schematic view of an ophthalmic applicator designed on the basis of Monte Carlo simulations;
FIG. 3 is a graph showing dose rate distributions of various radioisotopes with depths (Example 1(a) (not according to the present invention), Example 2 (d), Comparative Example 1 (b), Comparative Example 2 (c)).
FIG. 4 is a graph showing dose rate distributions of various radioisotopes with depths;and

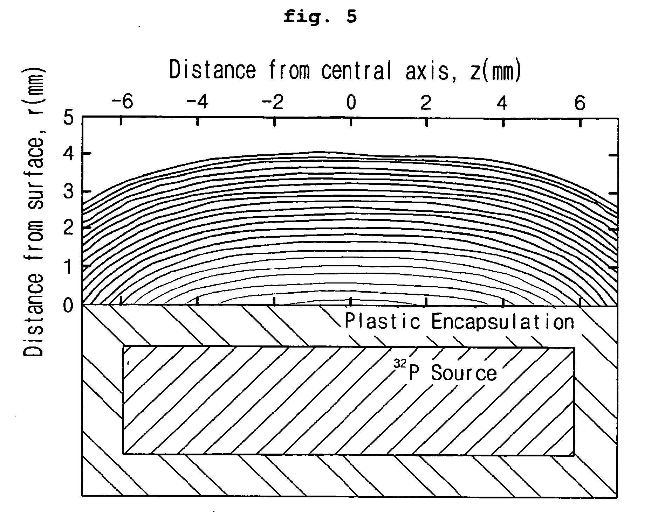

FIG. 5 is an isodose graph showing dose rate distributions of the embodiment.

### DESCRIPTION OF THE PREFERRED EMBODIMENTS

A detailed description will be given of the present invention with reference to the drawings.

The present invention pertains to an ophthalmic applicator for the treatment of pterygium or glaucoma using a combination of ³²P and ¹⁰³Pd, which shows far more even dose distribution, leading to an improvement in therapeutic effect on pterygium or glaucoma, and can correctly irradiate radiation onto a lesion, with higher safety for the eye lens than a conventional one using ⁹⁰Sr.

Disclosed herein is an applicator for the treatment of pterygium or glaucoma, comprising a source volume containing radioisotopes therein; a filter for controlling a radiation dose and radiation energy; and an encapsulator encompassing the source volume and the filter.

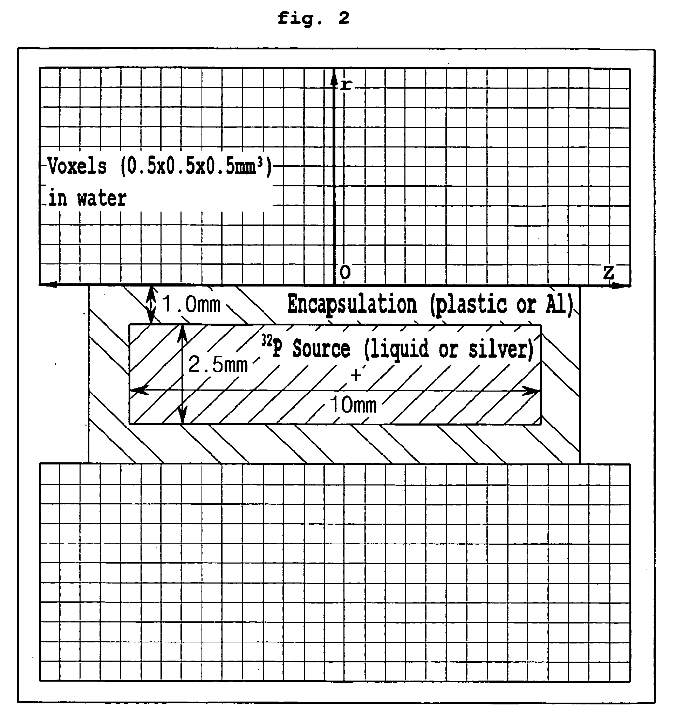

In the target irradiated with the radiation, the radiation dose is decreased in an exponential manner according to the depth. Doses of the applicator decrease with depth more rapidly than those of the ⁹⁰Sr applicator (Experimental Example 1 and FIG. 2).

Such a rapid decrease might be advantageous in radiotherapy for pterygium or glaucoma in consideration of the fact that the eye surface is to be intensively irradiated while the eye lens, which is spaced slightly apart from the eye surface, should receive a minimum dose.

Featuring the intensive accumulation of radiation doses in the target lesion and a rapid decrease in energy and radiation dose before the eye lens, therefore, the ophthalmic applicator using ³²P disclosed herein is superior in medicinal terms to the conventional ⁹⁰Sr applicator.

The present invention provides an applicator for the treatment of pterygium or glaucoma, comprising a source volume for containing a combination of ³²P and ¹⁰³Pd therein; a filter for controlling a radiation dose and radiation energy; and an encapsulator encompassing the source volume and the filter.

When an ophthalmic applicator employs a combination of ³²P and ¹⁰³Pd as a radiation source, the mixed radiation field lessens the stiff exponential decrease of the ³²P doses, which may be amplified by a geometrical error such as a setup of an applicator to the target lesion, leading to large variations in the dose delivered to the sclera.

Due to comparable half-lives of the two isotopes (14.2 days for ³²P vs. 16.9 days for ¹⁰³Pd), the emission ratio of electrons and photons can be maintained constant during the treatment, thereby allowing the available irradiation time period to be calculated accurately.

In accordance with the present invention, the ophthalmic applicator using a mixed radiation field of ¹⁰³Pd and ³²P is structured to allow radiation emission in such a way that the ³²P applicator is responsible for 80 % - 90 % of the total radiation dose while the ¹⁰³Pd applicator is responsible for 10 % - 20 % of the total radiation dose, correspondingly. When the dose of ³²P is outside of this range, the applicator does not confer the advantage of lessening the sharp decrease of the ³²P doses, amplified by a geometrical error. When the dose of ¹⁰³Pd is out of this range, an excessive radiation dose is delivered to the eye lens.

As long as it prevents the leakage of radioisotopes, any material may be used to construct the source volume therewith. Silver (Ag) may be a preferable material for the source volume.

In the ophthalmic applicator according to the present invention, the filter functions to control the radiation dose emitted from the radioisotope. That is, depending on the material and structure of the filter, the radiation dose delivered to the target lesion can be adjusted.

For the construction of the filter of the ophthalmic applicator according to the present invention, aluminum may be used as a material, as in a conventional one. as long as it prevents the leakage of radioisotopes, any material may be used to construct the source volume therewith.

The encapsulator in the ophthalmic applicator of the present invention functions to prevent the leakage of the radioisotopes ³²P and ¹⁰³Pd and is also required to reduce the attenuation of the radiation dose delivered to the target lesion to the greatest extent possible and to be thin and firm. The applicator of the present invention may take a conventional form or a modified form, which can be readily designed by those skilled in the art.

A better understanding of the present invention may be obtained through the following examples which are set forth to illustrate, but are not to be construed as the limit of the present invention.

Monte Carlo simulations were performed for the design and dosimetry of the ophthalmic applicator according to the present invention.

### EXAMPLE 1 (not according to the present invention): Design and Dosimetry Calculation of Ophthalmic Applicator Using ³²P

Using the Monte Carlo program MCNP5 code, an ophthalmic applicator using 32P alone was designed and calculated for dosimetry as follows[Yang Kyun Park, Sung-Joon Ye, Il Han Kim, Won Ryang Wee, Mee Kum Kim, Hyon Soo Han, Kwang-Jae Son, and Ul Jae Park, "Potential use of P-32 ophthalmic applicator: Monte Carlo simulations for design and dosimetry",pp. 1854-1858 Medical Physics, May 2008 Volume 35, Issue 5].

A ³²P ophthalmic applicator was designed to have a size similar to that of a conventional ⁹⁰Sr ophthalmic applicator. However, the design was focused on the structure and material of the encapsulator focus, with no filter volume imparted thereto, not only because ³²P is smaller in maximum beta energy than ⁹⁰Sr but also because the low-energy beta contribution of ³²P to the total dose is not large, unlike that of ⁹⁰Sr. The encapsulator was formed of a medical plastic material in order to ensure a sufficient encapsulation effect and minimum attenuation of the beta radiation and energy during delivery to a target lesion. The part to be brought into contact with a target lesion was designed to have a thickness of 0.5 mm. In order to calculate a dose distribution over depth in water, voxels having dimensions of 2.0 mm (width) x 2.0 mm (length) x 0.5 mm (thickness) were positioned according to depth, followed by the measurement of radiation doses at the depths. According to the Monte Carlo simulation, the transfer energy of beta radiation per unit of radioactivity (mCi or Bq) of each voxel was calculated per unit voxel weight to obtain a dose rate (cGy/s or Gy/s). From this, the radioactivity (mCi or Bq) necessary for a therapeutic dose or constant dose rate on a target lesion could be calculated.

### EXAMPLE 2: Design and Dosimetry Calculation of Ophthalmic Applicator Using ³²P and ¹⁰³Pd

Using the Monte Carlo code MCNP5, an ophthalmic applicator was designed and calculations for dosimetry were conducted therefor in the same manner as in Example 1, with the exception that ³²P was used in combination with ¹⁰³Pd instead of alone.

### COMPARATIVE EXAMPLE 1: Dosimetry of ⁹⁰Sr Applicator

Calculations for the use of a ⁹⁰Sr applicator for dosimetry were conducted using the Monte Carlo code MCNP5.

### COMPARATIVE EXAMPLE 2: Dosimetry of ¹⁰³Pd Applicator

Calculations for the use of a ¹⁰³Pd applicator were conducted for dosimetry using the Monte Carlo code MNCP5.

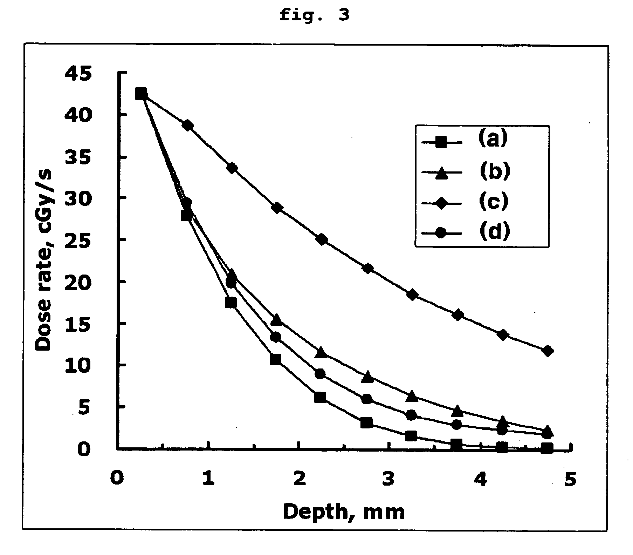

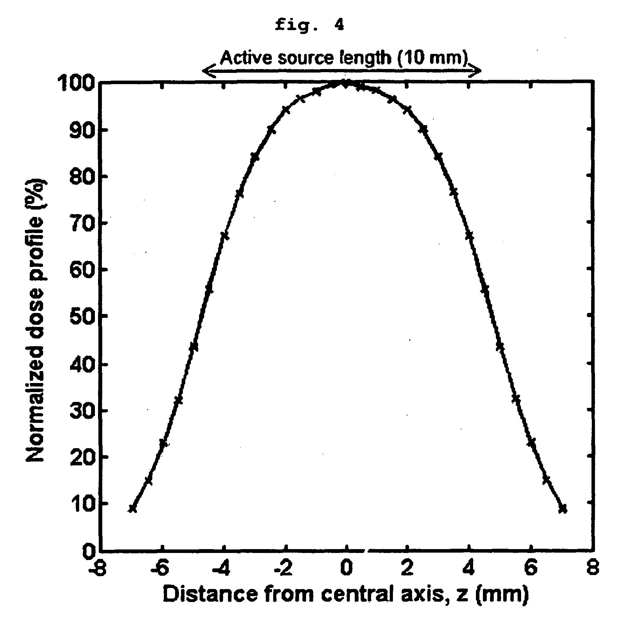

The calculation results of dosimetry obtained in Examples 1 and 2 and Comparative Examples 1 and 2 are summarized in Table 1 below and graphed in FIG. 3 and Fig. 4.

**TABLE 1**

| Dose Distribution (cGy/s) | | | | | | | | | | |
|---|---|---|---|---|---|---|---|---|---|---|
| Ex. Nos. | Depth (mm) | | | | | | | | | |
| | 0.25 | 0.75 | 1.25 | 1.75 | 2.25 | 2.75 | 3.25 | 3.75 | 4.25 | 4.75 |
| 1 | 42.5 | 26.6 | 16.3 | 9.7 | 5.7 | 3.1 1 | 1.6 | 0.8 | 0.3 | 0.1 |
| 2 | 42.5 | 28.4 | 18.9 | 12.6 | 8.6 | 6.0 | 4.2 | 3.13 | 2.4 | 1.9 |
| C. 1 | 42.5 | 28.6 | 20.9 | 15.5 | 11.7 | 8.8 | 6.4 | 4.8 | 3.5 | 2.4 |
| C. 2 | 42.5 | 28.6 | 33.7 | 29.0 | 25.2 | 21.7 | 18.5 | 16.1 | 14.0 | 12.0 |

The dose distributions shown in Table 1 and FIG. 3 are those which were normalized at a depth of 0.25 mm, where the dose rate was 42.5 cGy/s.

As seen in FIG. 3, dose rates of all applicators show exponential decrease with depth. Particularly, doses of Example 1 (a) decrease with depth more rapidly than those of Comparative Example 2 (c). The dose distributions of Example 2 (d) and Comparative Example 1 (b) are comparable within 3% at depths up to 0.75 mm, but the difference therebetween increases to as high as 17% at a depth of 1.25 mm. Such a rapid decrease is advantageous to the eye lens, which is spaced apart from the eye surface by 2 mm or more, meaning that only a small portion of the dose is delivered to the eye lens. That is, the applicator of Example 1 (a) can perform radiotherapy for pterygium, with less injury to the eye lens than that of Comparative Example 2 (c).

Also, the dose of the ophthalmic applicator of Comparative Example 1 (b) decreases with depth to a lesser extent, resulting in the penetration of an excess dose into the eye lens and thus injury to the eye lens.

In order to compensate for geometrical errors which lead to large variations in the dose delivered to the sclera due to the exponential decrease of dose with depth, a mixed radiation field consisting of 85% ³²P and 15% ¹⁰³Pd was employed in the ophthalmic applicator of Example 2 (d), whose dose distribution agreed with that of Comparative Example 1 within a 5% difference at depths up to 1.25 mm. In this case, the dose decrease rate of Example 2 (d) was lower than the exponential decrease rate of Example 1, allowing an improvement in the accuracy of irradiation time calculation.

In Table 2 are summarized radioactivities required to deliver a therapeutic radiation dose of 25 Gy to the eye surface for various time periods

**TABLE 2**

| Ex. No. | 1 hr | 10 hrs | 24 hrs |
|---|---|---|---|
| 1 | 19.8 (mCi) | 1.98(mCi) | 0.83(mCi) |
| 2 | 16.8 (mCi) + 1.4(Ci) | 1.68 (mCi ) + 140(mCi) | 0.7 (mCi ) + 58.3(mCi) |
| C. 1 | 0.67(mCi) | 0.067(mCi) | 0.028(mCi) |
| C. 2 | 9.3 x10³(mCi) | 931(mCi) | 388(mCi) |

The ophthalmic applicator of Example 1 was found to require a radioactivity of 19.8 mCi for a treatment time of 1 hr, 1.98 mCi for a treatment time of 10 hrs, and 0.83 mCi for a treatment time of 24 hrs.

In the ophthalmic applicator of Example 2, the fractioned radioactivity was required to be 16.8 mCi + 1.4 x10³ mCi for 1 hr, 1.68 mCi + 140 mCi for 10 hrs, and 0.7 mCi + 0.028 mCi for 24 hrs. These activities, required to deliver a therapeutic dose in a short time period, are producible in a pilot reactor.

As for the pure ¹⁰³Pd applicator (Comparative Example 2), the radioactivity required to deliver 25 Gy was measured to be 9.3 × 10³ mCi for 1 hr, 931 mCi for 10 hrs, and 388 mCi for 24 hrs. Thus, treatment with ¹⁰³Pd only is not plausible due to the large radioactivities required and large doses to the lens. Further, the applicator using only ¹⁰³Pd requires a longer dwelling time period than does the applicator using a mixed radiation field (Example 2), or is conducted in a fractioned treatment manner due to the required large radioactivity of ¹⁰³Pd. In either case of Example 1 and Comparative Example 2, however, the dose delivered to the sclera and lens should be almost the same as planned because their half-lives are similar (14.2 days for ³²P and 16.9 days for ¹⁰³Pd). In other words, the contributions of beta radiation and X-ray to the total dose are almost constant during the treatment.

Ensuring the formation of more ideal dose distributions than do the conventional ⁹⁰Sr ophthalmic applicators, as described hitherto, the ophthalmic applicator for the treatment of pterygium or glaucoma using a combination of ³²P and ¹⁰³Pd can promise both high therapeutic effects on pterygium or glaucoma and high safety effects on the eye lens. Further, ³²P and ¹⁰³Pd are easier to produce and treat than is ⁹⁰Sr, thereby allowing the radiotherapy to be useful.

## Claims

1. An ophthalmic applicator for treating a pterygium or glaucoma with a radioisotope comprising
a source volume containing the radioisotope therein;
a filter that functions to control the radiation dose emitted from the radioisotope; and
an encapsulator that encompasses the source volume and the filter,
**characterised in that** the radioisotope is a combination of ³²P and ¹⁰³Pd, and wherein the ³²P is responsible for 80 ∼ 90% of the total radiation dose while the ¹⁰³Pd is responsible for 20 ∼ 10% of the total radiation dose, correspondingly.

## Patentansprüche

1. Augenapplikator zur Behandlung eines Pterygiums oder eines Glaukoms mit einem Radioisotop, umfassend:
ein Quellvolumen, worin das Radioisotop enthalten ist;
einen Filter, der eine Kontrolle der von dem Radioisotop emittierten Strahlungsdosis bewirkt; und
eine Ummantelung, die das Ausgangsvolumen und den Filter umfasst, **dadurch gekennzeichnet, dass**
das Radioisotop eine Kombination aus ³²P und ¹⁰³Pd ist, und wobei das ³²P für 80-90% der gesamten Strahlungsdosis verantwortlich ist, während das ¹⁰³Pd entsprechend für 20-10% der gesamten Strahlungsdosis verantwortlich ist.

## Revendications

1. Applicateur ophtalmique pour traiter un ptérygion ou un glaucome avec un radio-isotope comprenant
un logement délimitant un volume source contenant le radio-isotope ;
un filtre dont la fonction est de contrôler la dose de rayonnement émis par le radio-isotope ; et
une capsule qui englobe le logement délimitant un volume source et le filtre, **caractérisé en ce que** le radio-isotope est une combinaison de ³²P et de ¹⁰³Pd, le ³²P étant responsable de 80 à 90% de la dose de rayonnement totale tandis que le ¹⁰³Pd est responsable de 10 à 20% de la dose de rayonnement totale, respectivement.
